(19) Europäisches Patentamt
European Patent Office
Office européen des brevets

(11) Publication number: 0 288 628 A1

# (12) EUROPEAN PATENT APPLICATION

(21) Application number: 87303897.0

(51) Int. Cl.[4]: C07D 241/52

(22) Date of filing: 30.04.87

(43) Date of publication of application: 02.11.88 Bulletin 88/44

(84) Designated Contracting States: CH DE ES FR LI

(71) Applicant: Chisso Corporation
6-32, Nakanoshima 3-chome Kita-ku
Osaka-shi Osaka-fu(JP)

(72) Inventor: Hiroshi, Harada
598, Furuichiba Ichiharashi
Chibaken(JP)
Inventor: Eisuke, Umeda
17, Higashi 2-chome Tatsumidai
Ichiharashi Chibaken(JP)
Inventor: Shinichi, Kawamura
5383-9, Goi Ichiharashi
Chibaken(JP)

(74) Representative: Lamb, John Baxter et al
MARKS & CLERK 57/60 Lincoln's Inn Fields
London WC2A 3LS(GB)

## (54) Process for producing 2-methyl-3-carbamido-quinoxaline-1, 4-di-n-oxides.

(57) A process for producing 2-methyl-3-carbamido-quinoxaline-1,4-di-N-oxides of the formula:

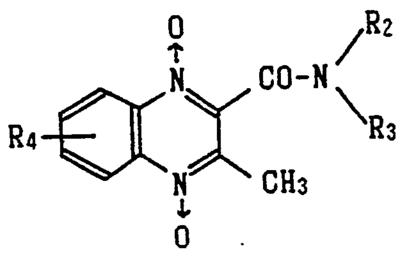

(1)

(in which each of $R_2$ and $R_3$ is a hydrogen atom or a straight or branched alkyl group optionally substituted with a hydroxy, lower alkoxy or alkoxycarbonyl group and; $R_4$ is a hydrogen atom or a lower alkyl or lower alkoxy group) comprises reacting an enamine of the formula:

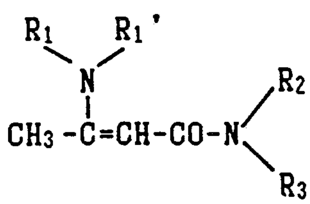

(2)

EP 0 288 628 A1

(in which each of $R_1$ and $R_1$, is a hydrogen atom or a straight or branched alkyl group optionally substituted with a hydroxy, lower alkoxy or alkoxycarbonyl group; and $R_2$ and $R_3$ have the meanings defined above) with a benzofuroxane of the formula:

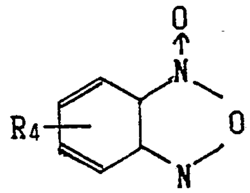

(in which $R_4$ has the meaning defined above) at 10 to 80°C in the presence of ammonia or a primary amine compound and in an organic solvent.

## PROCESS FOR PRODUCING 2-METHYL-3-CARBAMIDO-QUINOXALINE-1 4-DI-N-OXIDES

This invention relates to the preparation of 2-methyl-3-carbamido quinoxaline-1, 4-N-oxides.

Methods for the preparation of 2-methyl-3-carbamido-quinoxaline-1,4-di-N-oxides are disclosed in Japanese Patent Publication No. 24,988/1970 and Spanish Patent No. 476,376. However, the product obtained using these well-known methods contains impurities which are difficult to remove by recrystallization. Further, the yield is not satisfactory. Spanish Patent No. 495,817 and Austrian Patent No. 374,190 disclosed the preparation of 2-methyl-3-carbamido-quinoxaline-1,4-di-N-oxides by reaction between a benzofuroxane and a β-aminocrotonamide. This method is claimed to be better than the technique described in Austrian Patent No. 281,836 in that (a) it does not necessitate the isolation of an intermediate; (b) it does not require the use of diketene as a starting material; (c) the yield is higher; and (d) it does not require the use of ammonia.

However, even the method of Spanish patent No.495,817 and Austrian Patent No.374,190 leave room for improvement in the following respects:

(i) the product contains impurities which are difficult to remove; and

(ii) the use of β-aminocrotonamide compounds limit the range of compounds which may be manufactured.

The desired 2-methyl-3-carbamido-quinoxaline-1,4-di-N-oxides may be represented by the formula:

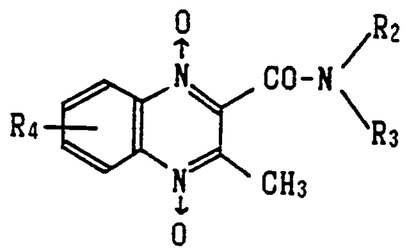

(1)

in which each of $R_2$ and $R_3$ is a hydrogen atom or a straight or branched alkyl group which may be substituted with a hydroxy, lower alkoxy or lower alkoxycarbonyl group; and $R_4$ is a hydrogen atom or a lower alkyl or lower alkoxy group.

Among the compounds of formula (1), the compound where each of $R_2$ and $R_4$ is hydrogen and $R_3$ is a 2-hydroxyethyl group:

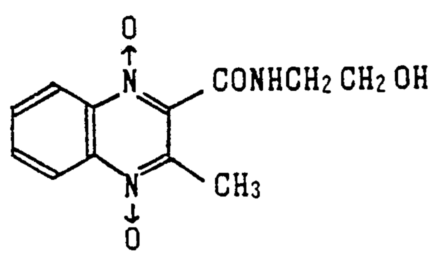

(1')

is known under the usual name of Olaquindox.

The method of the invention is especially suitable for the preparation of Olaquindox.

Olaquindox and related compounds are useful as veterinary medicines or as feed additives. In view of these uses, the effect of impurities in the compound may be significant.

Basically the invention provides a process for the preparation of a 2-methyl-3-carbamido-quinoxaline-1, 4-di-N-oxide of the formula:

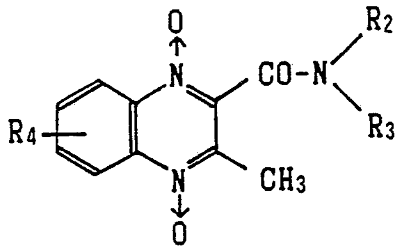

(1)

(in which $R_2$ and $R_3$ and $R_4$ have the meanings defined above) by reacting an enamine of the formula:

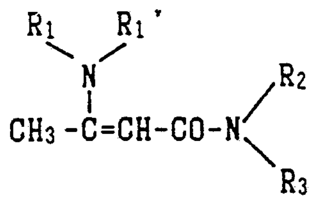

(2)

(in which $R_1$ and $R_1'$ are each a hydrogen atom or a straight a branched alkyl group optionally substituted with a hydroxy, lower alkoxy or alkoxycarbonyl group; and $R_2$ and $R_3$ have the meanings defined above), with a benzofuroxane derivative of the formula:

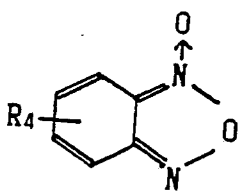

(3)

(in which $R_4$ has the meaning defined above) in the presence of ammonia or a primary amine and in an organic solvent, and at a temperature of from 10° to 80°C, preferably from 30 to 60°C.

In accordance with a first preferred embodiment of the invention the reaction is carried out by adding the benzofuroxane of formula (3) to a solution of the enamine in an organic solvent (preferably a polar organic solvent and especially a $C_1$-$C_6$ monohydric alcohol) which solution contains ammonia or primary amine.

In accordance with a second preferred embodiment of the invention the reaction is carried out in the presence of a mixed solvent system comprising a polar organic solvent and a non-polar organic solvent, the mixed polar solvent system being obtained by addition of the enamine in the polar organic solvent to a solution of the benzofuroxane in the non-polar organic solvent, which latter solvent also suitably contains ammonia or a primary amine.

The enamine of formula (2) can be readily produced in well-known manner by reaction of an acetoacetamide compound (for example acetoacetic dimethylamide, acetoacetic ethylamide, acetoacetic $\beta$-hydroxyethylamide, or acetoacetic $\beta$-methoxyethylamide), with an amine compound (for example ethylamine,n-butylamine or monoethanolamine) or ammonia. The benzofuroxane of formula (2) may, for example, be benzofuroxane, or 5-methyl-, 5-butyl, 5-methoxy-or 5-ethoxybenzofuroxane. The reaction relevant to the process of the invention can be represented by the following reaction scheme:

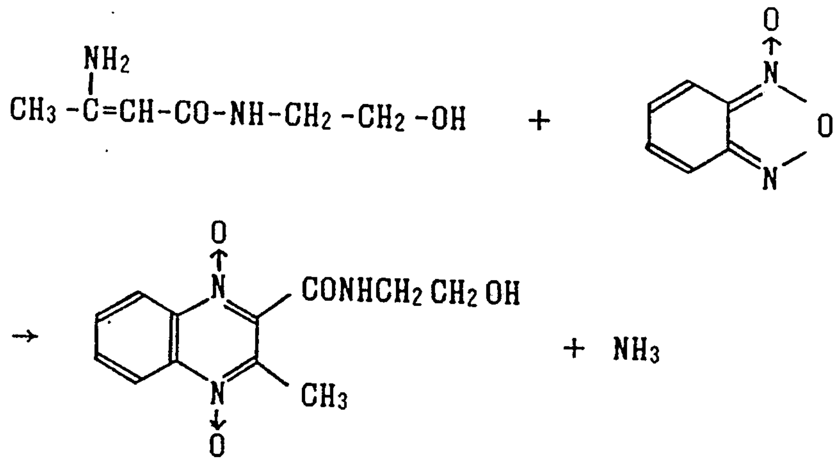

(5)

In the reaction exemplified by scheme (5), the molar ratio of benzofuroxane to enamine is suitably from 1:0.8 to 1:2.0, preferably from 1:1.0 to 1:1.5.

In the first embodiment of the invention the benzofuroxane is added to the enamine dissolved in an organic solvent in the presence of ammonia or a primary amine compound for subsequent reaction. In the second embodiment of the invention is, in place of acetoacetamide in known methods, the corresponding enamine compound is used, and two polar and nonpolar organic solvents, preferably two organic solvents of different polarity, are used to dissolve the two starting materials.

With respect to the solvent, the first embodiment of the invention uses only the enamine in the form of a solution in an organic solvent.

That is, the enamine is used in the form of a solution in an organic solvent in the first embodiment of the invention, whereas it is used in the form of a solution in a polar solvent, [for example an alcohol (preferably, methanol), acetonitrile, dimethylformamide, dimethyl suphoxide, ethyl acetate, tetrahydrofuran or dioxane)]; in the second embodiment of the invention. As polar organic solvent for the enamine solution, the solvent in which reaction has been carried between the amide and amine starting materials, may be used as it is. The concentration of the enamine in the solvent is not limited but will usually be from 0.1 to 20 moles/litre, preferably from 0.5 to 10 moles/litre.

In the second embodiment of the invention, the benzofuroxane is used in the form of the solution in a preferably non-polar solvent, for exmaple benzene, toluene or xylene.

The concentration of the benzofuroxane compound in the solvent is not limited, but is usually from 0.1 to 20 moles/litre, preferably from 0.5 to 10 moles/litre.

In a desirable mode of reaction, the solution of enamine in a polar organic solvent is placed in a reactor, and the solution of the benzofuroxane compound in a nonpolar organic solvent is added dropwise. The period of additional duration may vary (even a lump addition is tolerable) but is preferably 1 to 3 hours, preferably 2 to 20 hours after the completion of addition, preferably with stirring. The reaction is exothermic; the temperature is kept at 30 to 60°C by cooling if necessary, and then by heating if necessary. After reaction, the desired product precipitates as light yellow crystals. The product is isolated from the solvent, washed with a small amount of solvent, and dried.

2-Methyl-3-carbamido-quinoxaline-N-oxides obtained by the method of the invention contain almost no impurities which are difficult to remove by recrystallization, as compared with those synthetized by known methods.

Thus, crude Olaquindox obtained by various processes has the following properties.

| | Purity (wt%) | Impurity 1 (wt%) | Impurity 2 (wt%) |
|---|---|---|---|
| Known method | 99.06 | 1.36 | 0.78 |
| Present invention | 99.04 | 0.36 | 0 |
| First invention | 99.51 | 0.33 | 0 |

In the above table, Impurity 1 is removable, and Impurity 2 is difficult to remove by recrystallization. It is believed that the difference between Impurities 1 and 2 may affect very significantly the use (for example as a veterinary medicine) of this product.

The yield (based on starting benzofuroxane) of the desired 2-methyl-3-carbamido-quinoxaline-1,4-di-N-oxide, is markedly higher than that given by the known methods. Thus, whereas the yield from the known methods is about 73 to 74 weight % of theoretical, that from the process of the invention may be from 84 to 86%.

In addition, the performance and adjustment of the reaction is easy because the primary amine compound or ammonia can be preliminarily added to the enamine solution.

In order that the invention may be well understood, the following examples are given by way of illustration only.

Example 1.

First, 73.0g(1.2 moles) monoethanolamine is placed in 200 mℓ methanol of -10 to 0°C in a dry ice-acetone, and then 84.0g(1.0 mole) diketene is added dropwise with stirring, maintaining the temperature of the reaction mixture at -10 to 0 °C. After the completion of addition, the temperature of the reaction mixture is allowed to come to the room temperature, then raised to 35°C by heating, and stirred continuously for 2 hours at the same temperature.

While the obtained reaction mixture, a N-(2-hydroxyethyl)-acetoacetamide solution is cooled in a water bath, 51.0g (3.0 moles) ammonia gas is blown into for 50 minutes, maintaining the temperature of the reaction mixture at 35°C.

After the completion of ammonia gas blowing, the temperature of the reaction mixthre is raised to 45°C, and 136.0g (1.0 mole) benzofuroxane is added. The temperature of the reaction mixture is maintained at 45°C by cooling or heating for 7 hours(including the adding duration) with stirring. Then, the product precipitates as light yellow crystals. The reaction mixture is cooled, filtered with suction, washed with methanol, and dried to obtain N-(2-hydroxyethyl)-2-methyl-3-carbamido-quinoxaline-1,4-di-N-oxide. Yield: 228.3g (86.1% of theoretical), purity: 99.04%.

Comparative Example 1.

First, 73.0g(1.2 moles) monoethanolamine is placed in 200mℓ methanol of -10 to 0°C in a dry ice-acetone bath, and then 84.0g(1.0 mole) newely distilled diketene is added dropwise with stirring, maintaining the temperature of the reaction mixture at -10 to 0 °C. After the completion of addition,the temperature of the reaction mixture is allowed to come to the room temperature, then raised to 35°C by heating, and still stirred for 2 hours at the same temperature. To the obtained N-(2-hydroxyethyl)-acetoacetamide solution, 136.0g(1.0 mole)benzofuroxane is added dropwise, then about 3.0 mole ammonia is introduced, and the temperature of the reaction mixture is maintained below 45 °C by occasional cooling.

After the termination of the exothermic reaction, the reaction mixture is heated up to 40 to 45 °C, and

still stirred for 6 to 8 hours.

As a result, the reaction product precipitate as light yellow crystals. The reaction mixture is cooled, filtered with suction, washed with methanol, and dried to obtain N-(2-hydroxyethyl)-2-methyl-3-carbamido-quinoxaline-1,4-di-N-oxide. Yield: 193.8g(73.1% of theoretical), purity: 97.06%.

Example 2

First, 73.0g(1.2 moles) monoethanolamine is placed in 200 mℓ methanol of -10 to 0°C in a dry ice-acetone bath, and then 84.0g(1.0 mole) diketene is added dropwise with stirring, maintaining the temperature of the reaction mixture at -10 to 0 °C. After the completion of diketene addition, the temperature of the reaction mixture is allowed to come to the room temperature, then raised to 35°C by heating, and still stirred for 2 hours.

While the obtained reaction mixture, a N-(2-hydroxyethyl) -acetoacetamide solution, is cooled in a water bath, 51.0g (3.0 moles) ammonia gas is blown into the mixture, taking 50 minutes, maintaining the temperature at 35°C.

After the completion of ammonia gas blowing, the temperature of the reaction mixthre is raised to 45°C, 136.0g(1.0 mole) benzofuroxane dissolved in 400mℓ toluene is added.

The temperature of the reaction mixture is kept 45°C by cooling or heating with stirring for 14 hours.

Then, the reaction mixture is separated as light yellow crystals. The reaction mixture is cooled, filtered with suction, washed with methanol, and dried to N-(2-hydroxyethyl)-2-methyl-3-carbamido-quinoxaline-1,4-di-N-oxide. Yield: 226.2g(85.3%) of theoretical), purity: 99.51%.

Example 3 ~ 10

First, 1.2 moles of designated amine in Table 1 described below is placed in 200mℓ methanol (or toluene) of -10 ~ -5°C in a dry ice-acetone, and then 84.0g (1.0 mole) diketene is added dropweise with stirring, maintaining the temperature of the reaction mixture at -10~-5°C.

After the completion of addition,the temperature of the reaction mixture is allowed to come to the room temperature, then raised to 35 °C by heating, and stirred continuously for 2 hours at the same temperature (equation ①).

These yields and purities of thus obtained intermediates are shown in the Table 1.

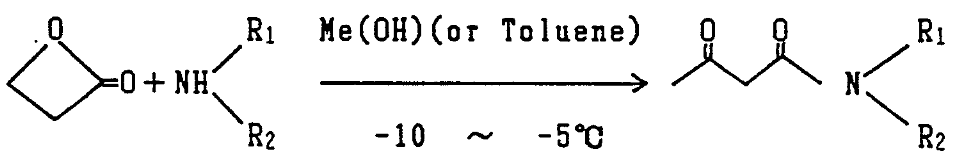

···①

While the obtained reaction mixture, a N-alkyl (or N-substituted alkyl)-acetoacetamide solution is cooled in water bath, 51.0 g (3.0 moles) ammonia gas is blown into for 20 minutes, maintaining the temperature of the reaction mixture at 35 °C (equation ②). These yields and purities of thus obtained enamine (in the form of reaction mixture) are shown in the Table 2.

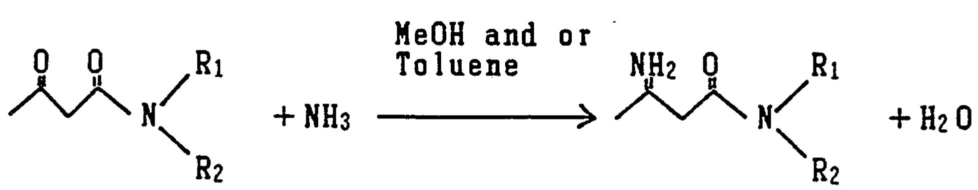

···②

After the completion of ammonia gas blowing, the temperature of the reaction mixthre is raised to 55°C, and 136.0g (1.0 mole) benzofuroxane or same amount of benzofuroxane dissolved 365mℓ of toluene is added. The temperature of the reaction mixture is maintained at 55°C by cooling or heating for 4 hours-

(including the adding duration) with stirring (equation ③).

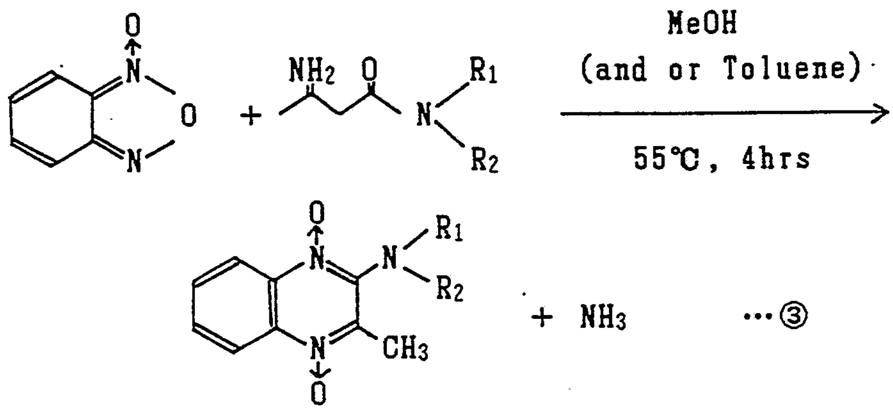


The reaction mixture is cooled,the product precipitates as light yellow crystals,then it is filtered with suction, washed with methanol, and dried to obtain 2-methyl-3-carbamido-quinoxaline-1,4-di-N-oxide. These yields and purities of these products are shown in the Table 3.

Table 1 Reaction of diketene and amines (equation ①)

| example NO. | kine of amines | solvent | N-alkyl-(acetoacetamide) | % yield, purity |
|---|---|---|---|---|
| 3 | propylamine | methanol | N-propyl- | 95 , 97 |
| 4 | butylamine | 〃 | N-butyl- | 96 , 97 |
| 5 | 3-methoxy-propylamine | 〃 | N-3-methoxy-propyl– | 93 , 98 |
| 6 | N-morpholine | 〃 | N-morpholino- | 95 , 98 |
| 7 | diethanol-amine | 〃 | N-(2-hydroxyethyl)- | 94 , 98 |
| 8 | monoethanol-amine | 〃 | N-(2-hydroxyethyl)- | 87 , 99 |
| 9 | 〃 | 〃 | 〃 | 87 , 98 |
| 10 | isopropyl-amine | toluene | N-isoprpyl- | 79 , 98 |

Table 2 Reaction of N-alkylacetacetamide and $NH_3$ (equation ②)

| example NO. | N-alkyl (aceto-actamide) | solvent | N-substituted β-amino-crotonamide) | % yield, purity |
|---|---|---|---|---|
| 3 | N-propyl | methanol | N-propyl- | 91 , 98 |
| 4 | N-butyl | 〃 | N-butyl- | 95 , 98 |
| 5 | N-3-methoxy | 〃 | N-3-methoxy propyl- | 93 , 98 |
| 6 | N-morpholino | 〃 | N-morpholino- | 93 , 99 |
| 7 | N-(2hydrox-yethyl)- | 〃 | N-(2-hydroxyethyl)- | 94 , 98 |
| 8 | N-(2hydrox-yethyl)- | 〃 | N-(2-hydroxyethyl)- | 87 , 99 |
| 9 | 〃 | 〃 | 〃 | 87 , 98 |
| 10 | N-i-propyl- | toluene | N-isopropyl- | 79 , 98 |

Table 3 Reaction of β-aminocrotonamide and benzofuroxane (equation ③)

| example NO. | -(β-amino-crotonamide) | solvent | N-alkyl- ( ✱ ) | % yield, purity |
|---|---|---|---|---|
| 3 | N-propyl- | methanol toluene | N-propyl- | 73 , 98 |
| 4 | N-butyl- | 〃 | N-butyl- | 87 , 98 |
| 5 | N-3-methoxy-propyl- | 〃 | N-3-methoxy-propyl | 86 , 98 |
| 6 | N-morpholino- | 〃 | N-morpholino- | 86 , 98 |
| 7 | N-(2-hydroxy-ethyl)- | 〃 | N-(2-hydroxyethyl)- | 90 , 98 |
| 8 | N-(2-hydroxy-ethyl)- | 〃 | N-(2-hydroxyethyl)- | 77 , 98 |
| 9 | 〃 | methanol | 〃 | 69 , 98 |
| 10 | N-i-propyl- | toluene | N-i-propyl- | 62 , 98 |

Note✱ 2-Methyl-3-carbamide-quinoxyaline-1,4-di-N-oxide

## Claims

1. A process for the production of a 2-methyl-3-carbamido-quinoxaline-1,4-di-N-oxide of the formula:

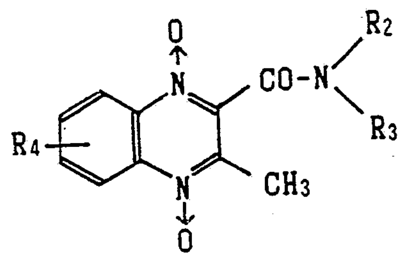

(1)

(in which each of $R_2$ and $R_3$ is a hydrogen atom or a straight or branched alkyl group optionally subsituted with a hydroxy, lower alkoxy or alkoxycarbonyl group and; $R_4$ is a hydrogen atom or a lower alkyl or lower alkoxy group) which comprises reacting an enamine of the formula:

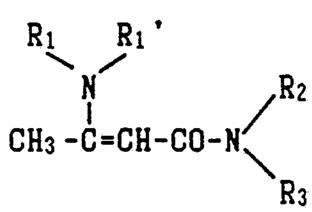

(2)

(in which each of $R_1$ and $R_1$, is a hydrogen atom or a straight or branched alkyl group optionally substituted with a hydroxy, lower alkoxy or alkoxycarbonyl group; and $R_2$ and $R_3$ have the meanings defined above) with a benzofuroxane of the formula:

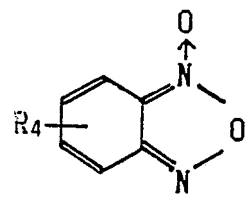

(3)

(in which $R_4$ has the meaning defined above) at 10 to 80°C in the presence of ammonia or a primary amine compound and in an organic solvent.

2. A process as claimed in claim 1 in which the benzofuroxane is added to a solution of the enamine in the organic solvention also containing ammonia or primary amine.

3. A process as claimed in claim 2 in which the organic solvent is a polar organic solvent, preferably a $C_1$-$C_6$ monohydric alcohol.

4. A process as claimed in claim 1 in which the organic solvent comprises a mixture of polar and non-polar organic solvents obtained by adding a solution of the benzofuroxane in the non-polar solvent to a solution of the enamine in the polar organic solution.

5. A process as claimed in claim 4 in which the polar solvent is a $C_1$-$C_6$ monohydric alcohol and the nonpolar solvent is benzene, toluene or xylene.

6. A process as claimed in any one of the preceding claims in which in the enamine, each of $R_1$ and $R_1$, is a hydrogen atom or hydroxy-substituted or unsubstituted, straight-chain alkyl group.

7. A process as claimed in any one of the preceding claims in which, in the benzofuroxane, $R_4$ is a hydrogen atom.

8. A process as claimed in any one of the preceding claims in which the enamine and the benzofuroxane are reacted together at 30 to 60°C.

European Patent Office

# EUROPEAN SEARCH REPORT

Application Number

EP 87 30 3897

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | AT-B- 374 190 (NEW CAMP S.R.L.) * claim * | 1 | C 07 D 241/52 |
| A | CHEMICAL ABSTRACTS OF JAPAN, vol. 96, no. 23, 7th June 1982, page 678, abstract no. 199728d, Columbus, Ohio, US; & ES - A - 49 5817 (Cat. D,A) | 1 | |
| A | GB-A-2 038 824 (LISAC, S.A.) * claim 1; page 1, left-hand column, formula scheme * & ES - A - 476 376 (Cat. D,A) | 1 | |
| A | DE-B-1 670 935 (BAYER AG) * claim 3 * | 1 | |
| A | DE-B-1 620 180 (RESEARCH CORP.) * claim; column 1, formula scheme * | 1 | |
| A | SYNTHESIS, no. 7, 1975, pages 415-422; K. LEY et al.: "Synthesen unter Verwendung von Benzofuroxan" * page 416, table 1, right-hand column; page 417, left-hand column, first two paragraphs * | 1 | TECHNICAL FIELDS SEARCHED (Int. Cl.4) C 07 D 241/00 |

The present search report has been drawn up for all claims

| Place of search | Date of completion of the search | Examiner |
|---|---|---|
| BERLIN | 24-12-1987 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document